# EUROPÄISCHE PATENTANMELDUNG

(11) **EP 1 170 277 A2**
(43) Veröffentlichungstag der Anmeldung: **09.01.2002**
(21) Anmeldenummer: 01111819.7
(22) Anmeldetag: 16.05.2001
(51) Int. Cl.: C07C 45/29, C07C 45/30, C08F 20/34, C08F 8/06

(54) **Verfahren zur Oxidation von Alkoholen mit homogen löslichen polymervergrösserten Stickstoffverbindungen als Katalysator**

(30) Priorität: 15.06.2000 DE 10029597
(71) Anmelder: Degussa Aktiengesellschaft, 40474 Düsseldorf (DE)
(72) Erfinder: Burkhardt, Olaf Dr., 2920 Kalmthout (BE); Wöltinger, Jens Dr., 63456 Hanau (DE); Karau, Andreas Dr., 67434 Neustad (DE); Philippe, Jean-Louis Dr., 63303 Dreieich (DE); Henniges, Hans Dr., 53111 Bonn (DE); Bommarius, Andreas Prof., 30327 Atlanta, GA (US); Krimmer, Hans-Peter Dr., 63128 Dietzenbach (DE); Drauz, Karlheinz Prof., 63579 Freigericht (DE)

(57) **Zusammenfassung**

Die vorliegende Erfindung betrifft ein Verfahren zur Oxidation von Alkoholen unter Verwendung eines Oxidationsmittels und katalytischer Mengen von homogen löslichen polymervergrößerten Nitroxylderivaten, welche durch Copolymerisation einer Mischung aufweisend eine Verbindung (I) 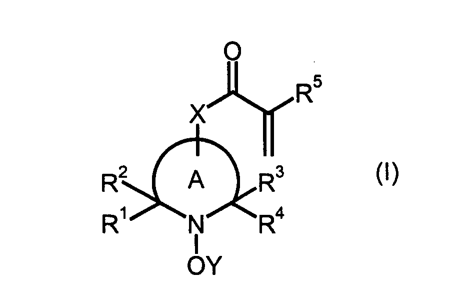 gewonnen werden. Das gegenständliche Verfahren kann wie mit monomerem TEMPO in einem Zweiphasensystem ausgeführt werden und liefert die Oxidationsprodukte in kurzer Zeit und hohen Ausbeuten. Die so erhaltenen Produkte eigenen sich für die Weiterverarbeitung zu bioaktiven Wirkstoffen.

## Beschreibung

Die vorliegende Erfindung ist auf ein Verfahren zur Oxidation von Alkoholen mithilfe homogen löslicher polymervergrößerter Stickstoffverbindungen als Katalysatoren gerichtet.

Insbesondere betrifft die Erfindung ein Verfahren, bei dem als Katalysatoren Verbindungen eingesetzt werden, welche durch Copolymerisation einer Mischung aufweisend
A: 0,1-100, vorzugsweise 1-20 Gew.-% einer Verbindung (I) 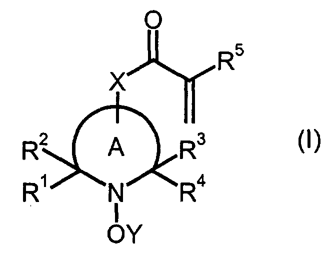
B: 0-99,9, vorzugsweise 80-99 Gew.-% (Meth)acrylsäureester
C: 0-80, vorzugsweise 1-20 Gew.-% weiterer α,β-ungesättigter Verbindungen, wie HEMA oder EGDMA, erhältlich sind.

Die Oxidation von Alkoholen stellt in der organischen Synthese eine wichtige Transformation zum Erhalt von Aldehyden, Ketonen oder Säuren dar. Diese wiederum dienen vorteilhafterweise, sofern nicht selbst als Zeilmolekül gedacht, zur weiteren Umwandlung in Folgeprodukte, da sie sehr leicht nucleophilen Additionsreaktionen zugänglich sind. Ihnen kommt somit gerade auch in der technischen Herstellung von bioaktiven Molekülen häufig eine Schlüsselrolle im Syntheseweg zu.

Die Oxidation von sekundären und primären Alkoholen zu Aldehyden und Ketonen mit N-Sauerstoffverbindungen des 2,2,6,6-tetramethyl-4-piperidin (TEMPO) in Gegenwart von Oxidantien wie m-CPBA, Hypochlorit/bromit-Lösung oder K₃Fe(CN)₆ ist seit langem bekannt (J. Org. Chem. 1987, 52, 2559-62; ibid 1975, 40, 1860; Synthesis 1966, 1153).

Darüber hinaus sind schon polymervergrößerte TEMPO-Radikale synthetisiert worden mit dem Zweck, diese als UV-Stabilisatoren in Polymermischungen einzuarbeiten (DE 2748362; L. Wenzhong, Polym. Degra. and Stab. 1991, 31, 353-364).
Von Endo et al. wurden u.a. teilweise lösliche polymervergrößerte TEMPO-Verbindungen in Alkoholoxidationen eingesetzt (Journal of Polymer Science: Polymer Chemistry Edition, Vol. 23, 2487-94 (1985)). Es zeigte sich jedoch, daß die unlöslichen Oxidationskatalysatoren des dort vorgestellten Typs die besseren waren.

Aufgabe der vorliegenden Erfindung war deshalb die Angabe eines weiteren Verfahrens zur Oxidation von Alkoholen in Gegenwart von Oxidationskatalysatoren auf Basis von homogen löslichen polymervergrößerten Stickstoffverbindungen anzugeben. Insbesondere sollte dieses Verfahren im technischen Maßstab anwendbar also im Hinblick auf ökonomische und ökologische Gesichtspunkte vorteilhaft sein.

Diese Aufgabe wird durch ein Verfahren gemäß geltendem Anspruch 1 erfüllt. Ansprüche 2 bis 6 sind auf weitere bevorzugte Ausgestaltungen des erfindungsgemäßen Verfahrens gerichtet. Anspruch 7 schützt eine bevorzugte Verwendung der erfindungsgemäß hergestellten Verbindungen.

Dadurch, daß man bei einem Verfahren zur Oxidation von Alkoholen ein Oxidationsmittel und katalytische Mengen von homogen löslichen polymervergrößerten Nitroxylderivaten verwendet, wobei diese durch Copolymerisation einer Mischung aufweisend
A: 0,1-100, vorzugsweise 1-20 Gew.-% einer Verbindung (I) 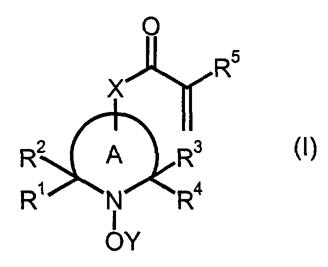 worin Y = • oder H ist,
   A ist ein 5 bis 8 gliedriger Ring, der 0-3 weitere Heteroatome, wie N, O, S, und 0-3 weitere Reste, wie (C₁-C₈)-Alkyl, (C₁-C₈)-Alkoxy, Halogene, aufweisen kann,
   R¹, R², R³, R⁴ sind unabhängig voneinander (C₁-C₈)-Alkyl, (C₆-C₁₈)-Aryl, (C₇-C₁₉)-Aralkyl, (C₃-C₈)-Cycloalkyl,
      oder R¹ und R² und/oder R³ und R⁴ oder R¹ und R³ und/oder R² und R⁴ sind über eine (C₂-C₈)-Alkylenbrücke miteinander verbunden,
   R⁵ ist H oder Methyl,
   X ist O, NH, NR¹,
B: 0-99,9, vorzugsweise 80-99 Gew.-% (Meth)acrylsäureester
C: 0-80, vorzugsweise 1-20 Gew.-% weiterer α,β-ungesättigter Verbindungen, wie HEMA oder EGDMA, gewonnen werden, gelangt man in überraschend einfacher und kostengünstiger Weise zu den gewünschten Alkoholoxidationsprodukten, vorzugsweise den Aldehyden und Ketonen. Erfindungsgemäß ist die Oxidationsreaktion innerhalb weniger Minuten abgeschlossen und die Mischung kann aufgearbeitet werden, wobei einerseits der Katalysator hervorragend abgetrennt und wiederverwendet werden kann und andererseits die Ausbeuten an Oxidationsprodukt nahezu quantitativ sind. Durch die Wiederverwendbarkeit des Katalysators können darüber hinaus die Synthesekosten niedriger gehalten werden.

In einer bevorzugten Ausführungsform sind die Reste R¹ - R⁴ = Methyl, R⁵ = Methyl oder H, X = O, NH, wobei A einen Piperidinring darstellt.

Besonders bevorzugt ist ein Verfahren, bei dem das polymervergrößerte Nitroxylderivat der Formel (II) 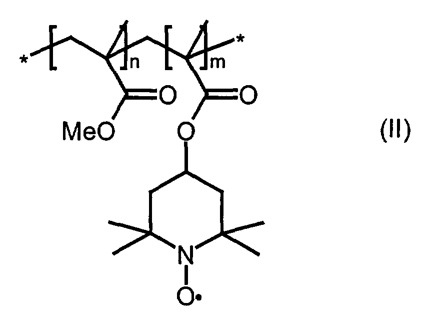 mit einem Verhältnis von n/m von 1-100, vorzugsweise 1-50, und einem mittleren Molekulargewicht von 1-200 kDa, vorzugsweise 10-100 kDa, eingesetzt wird.

Als weitere α,β-ungesättigte Verbindungen des Typs C können insbesondere solche Monomere dienen, welche helfen, die Löslichkeitseigenschaften des Polymers zu veränderen bzw. an das zu verwendende Lösungsmittelsystem ideal anzupassen. Diese Monomere können weiterhin vernetzend wirken. Dadurch wird erreicht, daß einzelne Polymerstränge miteinander verbunden werden, was wiederum die Löslichkeitseigenschaften und die Sekundärstruktur des Polymerrückrats und damit indirekt die Reaktivität des Katalysators ganz wesentlich beeinflussen kann. Als weitere Verbindungen können vorzugsweise die Monomeren der Komponente A) und B) des Polymerisats iii) in der DE 19734360 in Frage kommen. Ganz besonders bevorzugt ist der Einsatz von HEMA oder EGDMA in dieser Hinsicht.

Als Oxidationsmittel können im Prinzip die für den Fachmann für diese Reaktion in Frage kommenden Substanzen verwendet werden. Vorzugsweise sind dies K₃Fe(CN)₃ oder wäßrige NaOCl-Lösung. Bevorzugt ist wäßrige NaOCl-Lösung, da sie günstiger ist und keine Cyanid-Problematik - gerade im großtechnischen Maßstab - mit sich bringt.

Die erfindungsgemäße Oxidation wird prinzipiell entsprechend der mit monomeren TEMPO durchgeführt, vorzugsweise im Zweiphasensystem aus organischem und wäßrigem Lösungmittel. Als bevorzugte organische Lösungsmittel können Ethylacetat, Acetonitril, Dichlormethan oder Benzonitril dienen. Ganz besonders bevorzugt ist Ethylacetat, Acetonitril.

Oxidationsmittel, polymervergrößertes Nitroxylderivat der Formel (I), vorzugsweise das der Formel (II), wird in dem gewählten Zweiphasensystem gelöst. In der wäßrigen Phase wird der pH-Wert so eingestellt, daß die Oxidation bei einem pH von 6-13, vorzugsweise 9-10, abläuft. Zur Einstellung des pH-Wertes wird bevorzugt Natriumcarbonat verwendet. Es können jedoch auch alle anderen dem Fachmann für diesen Zweck in Frage kommende Basen verwendet werden, wie z.B. Kaliumcarbonat, Natriumhydrogencarbonat, Natriumhydrogenphosphat. Anschließend kann der Alkohol zu dem Gemisch gegeben werden. Die Reaktion ist meist in wenigen Minuten quantitativ abgeschlossen.

Die Reaktion wird vorzugsweise bei Temperaturen von -20°C-80°C, bevorzugt 0 - 30°C, durchgeführt. Nach Beendigung der Reaktion kann diese nach dem Fachmann bekannten Verfahren aufgearbeitet werden.

Arbeitet man im Zweiphasensystem, so wird die organische produkthaltige und ggf. katalysatorhaltige Phase von der wäßrigen getrennt. Ein besonderer Vorteil des erfindungsgemäßen Verfahrens ist, daß der polymervergrößerte Katalysator nach vollendeter Reaktion leicht aus der organischen Phase wiedergewonnen werden kann und so für einen weiteren Oxidationscyclus zur Verfügung steht. Dies kann durch Filtration mittels einer Ultra-/Nanofiltrationsmembran erfolgen oder durch Ausfällen unter Zugabe geeigneter Lösungsmittel, vorzugsweise Alkoholen wie Methanol oder Ethanol, Petrolether, Hexan oder Diethylether.

Ganz besonders bevorzugt ist aber die Anwendung des Verfahrens in einem Membranreaktor. Dadurch können die normalerweise im Batchverfahren durchgeführten Synthesen quasikontinuierlich oder kontinuierlich erfolgen, was aus Kostensicht für ein technisches Verfahren besonders vorteilhaft erscheint. Die Anwendung des erfindungsgemäßen Verfahrens im Membranreaktor läuft analog den im Stand der Technik beschriebenen Verfahren (T. Müller, J. Mol. Cat. A: Chem. 1997, 116, 39-42; DE 199 10 691.6; Wandrey et al., Tetrahedron Asymmetry 1999, 10, 923-928). Der Membranreaktor kann dabei als Cross-Flow- oder als Dead-End-Filtrationsmodul agieren (DE 19947505.9 sowie DE 19910691.6 oder Engineering processes for Bioseparations" Edited by: Laurence R. Weatherley Seiten: 135-165; Butterworth-Heinemann, 1994; ISBN: 0 7506 1936 8).

Ein weiterer Aspekt der Erfindung beschäftigt sich mit der Verwendung der erfindungsgemäß hergestellten Oxidationsprodukte in der organischen Synthese, vorzugsweise zur Herstellung von bioaktiven Verbindungen.

Das gegenständliche Verfahren erlaubt eine einfache, im technischen Maßstab gut durchzuführende Oxidation von Alkoholen zu den entsprechend gewünschten Derivaten. Dabei ergeben sich - verglichen mit den bekannten unlöslichen polymervergrößerten Spezies - überraschend kurze Reaktionszeiten. Dieses und die Tatsache, daß der Katalysator leicht recycliert und wieder eingesetzt werden kann, sind mithin ursächlich dafür, daß das beanspruchte Verfahren sehr gut für den technischen Einsatz geeignet ist. Des weiteren ist eine ausgezeichnete Selektivität zugunsten primärer Alkohole festzustellen, falls im Substrat, oder im Reaktionsgemisch, sowohl primäre als auch sekundäre Alkohole vorhanden sind.

Als (C₁-C₈)-Alkyl sind anzusehen Methyl, Ethyl, *n*-Propyl, Isopropyl, *n*-Butyl, Isobutyl, sec-Butyl, tert-Butyl, Pentyl, Hexyl, Heptyl oder Octyl samt aller Bindungsisomeren.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste. Diese können einfach oder mehrfach mit (C₁-C₈)-Alkoxy, (C₁-C₈)-Haloalkyl, OH, Cl, NH₂, NO₂ substituiert sein. Außerdem kann der Rest ein oder mehrere Heteroatome wie N, O, S aufweisen.

(C₁-C₈)-Alkoxy ist ein über ein Sauerstoffatom an das betrachtete Molekül gebundener (C₁-C₈)-Alkyl-Rest.

Ein (C₇-C₁₉)-Aralkylrest ist ein über einen (C₁-C₈)-Alkylrest an das Molekül gebundener (C₆-C₁₈)-Arylrest.

Unter dem Begriff Acrylat wird im Rahmen der Erfindung auch der Begriff Methacrylat verstanden.

(C₁-C₈)-Haloalkyl ist ein mit einem oder mehreren Halogenatomen substituierter (C₁-C₈)-Alkyl-Rest. Als Halogenatome kommen insbesondere Chlor und Fluor in Betracht.

Unter dem Begriff (C₂-C₈)-Alkylen-Kette ist ein (C₂-C₈)-Alkylrest zu verstehen, der über zwei verschiedene C-Atome an das betreffende Molekül gebunden ist. Dieser kann einfach oder mehrfach mit (C₁-C₈) -Alkoxy, (C₁-C₈)-Haloalkyl, OH, Halogen, NH₂, NO₂, SH, S-(C₁-C₈)-Alkyl substituiert sein.

Unter (C₃-C₈)-Cycloalkyl versteht man Cyclopropyl, Cyclobutyl, Cyclopentyl, Cyclohexyl bzw. Cycloheptyl oder Cyclooctylreste.

Halogen ist Fluor, Chlor, Brom, Iod.

Im Rahmen der Erfindung wird unter Membranreaktor jedwedes Reaktionsgefäß verstanden, bei dem der Katalysator in einem Reaktor eingeschlossen wird, während niedermolekulare Stoffe dem Reaktor zugeführt werden oder ihn verlassen können. Dabei kann die Membran direkt in den Reaktionsraum integriert werden oder außerhalb in einem separaten Filtrationsmodul eingebaut sein, bei der die Reaktionslösung kontinuierlich oder intermittierend durch das Filtrationsmodul strömt und das Retentat in den Reaktor zurückgeführt wird. Geeignete Ausführungsformen sind u.a. in der WO98/22415 und in Wandrey et al. in Jahrbuch 1998, Verfahrenstechnik und Chemieingenieurwesen, VDI S. 151ff.; Wandrey et al. in Applied Homogeneous Catalysis with Organometallic Compounds, Vol. 2, VCH 1996, S.832 ff.; Kragl et al., Angew. Chem. 1996, 6, 684f. beschrieben.

### Beispiele:

### 1.1. Polymerisation von 4-Methacryloyloxy-2,2,6,6-teramethylpiperidin mit Methylmethacrylat

### Reaktionsgleichung:

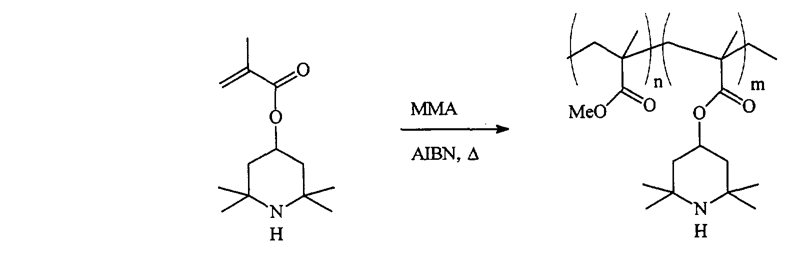

### Chemikalien :

4-Methacryloyloxy-2,2,6,6-tetramethylpiperidin M=225 g/mol: 4,05 g (18 mmol)
Methylmethacrylat (MMA) M=100,12 g/mol: 18,02 g (180 mmol)
2,2'-Azobis(2-methylpropionitril) (AIBN) M=164,21 g/mol: 0,325 g (1,98 mmol)
Isobutylmethylketon: 40 ml
Petrolether: 550 ml

### Durchführung :

18,02g MMA, 20ml Isobutylmethylketon, 4,05 g 4-Methacryloyloxy-2,2,6,6-tetramethylpiperidin und 0,325 g AIBN werden im 100 ml Dreihalskolben unter Stickstoff auf 80°C erwärmt. Der Ansatz wird 2,5 h bei 80°C, anschließend über Nacht bei Raumtemperatur unter Stickstoff nachgerührt. Das zähflüssige Reaktionsgemisch wird mit 20 ml Isobutylmethylketon verdünnt und auf 80°C erwärmt. Die warme Lösung wird in 500 ml Petrolether langsam eingegossen. Ein weisser Niederschlag bildet sich. Die Suspension wird 2h nachgerührt. Der Niedrschlag wird abfiltriert, und der Filterkuchen wird mit 50 ml Petrolether nachgewaschen. Das Produkt, ein weisses Pulver, wird bei 40°C im Vakuum getrocknet. NMR-Spektren bestätigen das gewünschte Produkt mit einem Verhältnis n/m ~10
Ausbeute: 21,2 g (96,1 % d. Th.)
GPC: Mn = 46000 g/mol Mw = 62000 g/mol

Bei einem analogen Versuch mit 2 mol% AIBN wird ein Polymer mit Mn = 21250 g/mol und Mw= 41300 g/mol erhalten.

### 1.2. Umsetzung von polymeren 4-Methacryloyloxy-2,2,6,6-teramethylpiperidin mit Wasserstoffperoxid zum entsprechenden 4-Methacryloyloxy-2,2,6,6-teramethylpiperidin-1-oxyl

### Reaktionsgleichung:

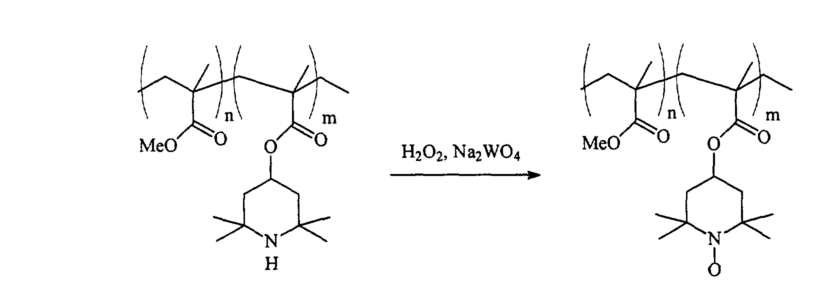

### Chemikalien :

Polymeres 4-Methacryloyloxy-2,2,6,6-tetramethylpiperidin (Mn = 21250 g/mol und Mw = 41300 g/mol) M=1226 g/mol: 10,17 g (8,3 mmol)
Wasserstoffperoxid (30%) M=34 g/mol: 1,36 g (12 mmol)
Natriumwolframat-Dihydrat M=329,9 g/mol: 0,016 g (0,05 mmol)
Ethylendiamintetraessigsäure M=292,3 g/mol: 0,026 g (0,09 mmol)
Petrolether: 750 ml
Methanol: 250 ml
Isobutylmethylketon: 50 ml
Tetrahydrofuran: 50 ml
VE-Wasser: 800 ml

### Durchführung :

10,17g polymeres 4-Methacryloyloxy-2,2,6,6-tetramethylpiperidin, 0,026g Ethylendiamintetraessigsäure und 0,016g Natriumwolframat-Dihydrat werden im 500ml Dreihalskolben in einem Gemisch aus 250ml Methanol und 50ml Isobutylmethylketon gelöst. Unter Rühren werden 1,36g Wasserstoffperoxid (30%) langsam zugesetzt. Es wird 3 Tage bei Raumtemperatur nachgerührt. Anschließend werden am Rotationsverdampfer ca. 200ml Lösungsmittel bei 40°C/400 mbar abdestilliert. Der Rückstand (gelb-orange) wird in 700ml Petrolether (auf 5°C gekühlt) eingetropft. Es wird 2h bei 0°C-5°C nachgerührt - hierbei bildet sich ein klebriger Niederschlag. Den klebrigen Niederschlag lässt man absitzen - der überstehende Petrolether wird abdekantiert. Der Rückstand wird in 50ml Tetrahydrofuran gelöst und in 700ml VE-Wasser bei Raumtemperatur eingetropft - Ein weisser Niederschlag bildet sich. Die Suspension wird über Nacht nachgerührt, anschließend filteriert, und der Filterkuchen mit 100ml VE-Wasser nachgewaschen. Das Produkt, ein rosa Pulver, wird bei 40°C im Vakuum getrocknet. NMR und UV bestätigen die gewünschte Struktur.
Ausbeute: 9,7 g (94,2 % d. Th.)
GPC: Mn = 19350 g/mol; Mw = 41100 g/mol

Ein analog durchgeführter Ansatz mit einem höher polymeren Edukt (Mn = 46000 g/mol und Mw= 62000 g/mol ) führt zu einem analogen Produkt mit Mn = 40250 g/mol und Mw = 63750 g/mol.

### 1.3. Oxidation von 1-Hexanol zu Hexanal in Gegenwart von polymeren 4-Methacryloyloxy-2,2,6,6-teramethylpiperidin-1-oxyl

### Reaktionsgleichung:

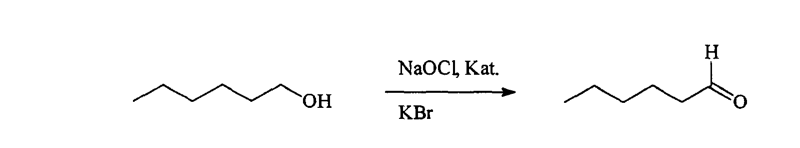

### Chemikalien :

Polymeres 4-Methacryloyloxy-2,2,6,6-tetramethylpiperidin-1-oxyl (Mn = 40250 g/mol und Mw = 63750 g/mol) M=1241 g/mol: 0,62 g (0,5 mmol)
1-Hexanol M=102,18 g/mol: 5,11 g (50 mmol)
Natriumhypochloridlösung (7,49% NaOCl) M=74,44 g/mol:
   54,61 g (55 mmol) (mit festem Natriumhydrogencarbonat auf pH 9,5 einstellen)
Kaliumbromidlösung (0,5M) M=119,01 g/mol: 10 ml (5 mmol)
Natriumthiosulfatlösung (10%): 125 ml
Dichlormethan: 80 ml

### Durchführung :

0,62g polymeres 4-Methacryloyloxy-2,2,6,6-tetramethylpiperidin-1-oxyl, 5,11g 1-Hexanol, 20ml Dichlormethan und 10 ml 0,5M Kaliumbromidlösung werden im 100ml Dreihalskolben vorgelegt und auf -10°C gekühlt. 54,61g Natriumhypochloridlösung (7,49% NaOCl ,mit festem Natriumhydrogencarbonat auf pH 9,5 eingestellt) werden innerhalb von 20 Minuten zugetropft. Die Reaktion ist exotherm, und die Reaktionstemperatur wird durch Kühlen zwischen 0°C und 5°C gehalten. Es wird 10 Minuten bei 0°C - 5°C nachgerührt. Die wässrige Phase wird im Scheidetrichter abgetrennt und 3 mal mit je 20ml Dichlormethan ausgeschüttelt. Die organischen Phasen werden vereinigt und 2 mal mit je 25ml - und 1mal mit 75ml Natriumthiosulfatlösung (10%) gewaschen. Die org. Phase wird mit Magnesiumsulfat getrocknet. Das Magnesiumsulfat wird mittels Filtration wieder entfernt. Das Filtrat wird am Rotationsverdampfer bei 35°C - 40°C / 400 mbar eingeengt. Das erhaltene Rohprodukt wird direkt analysiert.

Die NMR-Analyse bestätigt das Wunschprodukt und zeigt keinerlei Hinweise auf Restalkohol oder Säuren.
Ausbeute: 4,64 g = 72,8 % d. Th.

### 1.4. Oxidation von 2-Hexanol zu 2-Hexanon in Gegenwart von polymeren 4-Methacryloyloxy-2,2,6,6-teramethylpiperidin-1-oxyl

### Reaktionsgleichung:

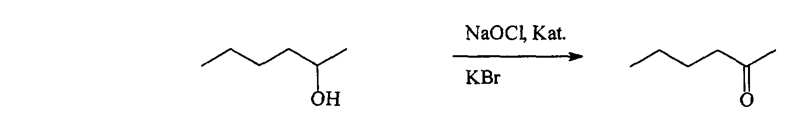

### Chemikalien:

Polymeres 4-Methacryloyloxy-2,2,6,6-tetramethylpiperidin-1-oxyl (Mn = 40250 g/mol und Mw = 63750 g/mol) M=1241 g/mol: 0,62 g (0,5 mmol)
2-Hexanol M=102,18 g/mol: 5,11 g (50 mmol)
Natriumhypochloridlösung (7,49% NaOCl) M=74,44 g/mol: 54,61 g (55 mmol)
   (mit festem Natriumhydrogencarbonat auf pH 9,5 einstellen)
Kaliumbromidlösung (0,5M) M=119,01 g/mol: 10 ml (5 mmol)
Natriumthiosulfatlösung (10%): 125 ml
Dichlormethan: 80 ml

### Durchführung :

0,62g polymeres 4-Methacryloyloxy-2, 2, 6, 6-tetramethylpiperidin-1-oxyl, 5,11g 2-Hexanol, 20ml Dichlormethan und 10 ml 0,5M Kaliumbromidlösung werden im 100ml Dreihalskolben vorgelegt und auf -10°C gekühlt. 54,61g Natriumhypochloridlösung (7,49% NaOCl ,mit festem Natriumhydrogencarbonat auf pH 9,5 eingestellt) werden innerhalb von 20 Minuten zugetropft. Die Reaktion ist exotherm, und die Reaktionstemperatur wird durch Kühlen zwischen 0°C und 5°C gehalten. Es wird 10 Minuten bei 0°C - 5°C nachgerührt. Die wässrige Phase wird im Scheidetrichter abgetrennt und 3 mal mit je 20ml Dichlormethan ausgeschüttelt. Die organischen Phasen werden vereinigt und 2 mal mit je 25ml - und 1mal mit 75ml Natriumthiosulfatlösung (10%) gewaschen. Die org. Phase wird mit Magnesiumsulfat getrocknet. Das Magnesiumsulfat wird mittels Filtration wieder entfernt. Das Filtrat wird am Rotationsverdampfer bei 35°C - 40°C / 400 mbar eingeengt. Das erhaltene Rohprodukt wird direkt analysiert.

Die NMR-Analyse bestätigt das Wunschprodukt und zeigt keinerlei Hinweise auf Restalkohol oder Säuren.
Ausbeute: 4,58 g = 81,8 % d. Th.

## Patentansprüche

1. Verfahren zur Oxidation von Alkoholen unter Verwendung eines Oxidationsmittels und katalytischer Mengen von homogen löslichen polymervergrößerten Nitroxylderivaten erhältlich durch Copolymerisation einer Mischung aufweisend
A: 0,1-100, vorzugsweise 1-20 Gew.-% einer Verbindung (I) 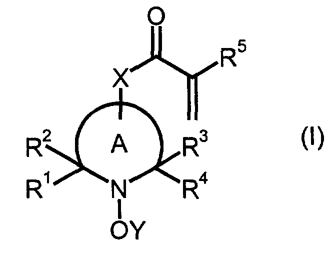 worin Y = • oder H ist,
A ist ein 5 bis 8 gliedriger Ring, der 0-3 weitere Heteroatome, wie N, O, S, und 0-3 weitere Reste, wie (C₁-C₈)-Alkyl, (C₁-C₈)-Alkoxy, Halogene, aufweisen kann,
R¹, R², R³, R⁴ sind unabhängig voneinander (C₁-C₈)-Alkyl, (C₆-C₁₈) -Aryl, (C₇-C₁₉)-Aralkyl, (C₃-C₈)-Cycloalkyl,
oder R¹ und R² und/oder R³ und R⁴ oder R¹ und R³ und/oder R² und R⁴ sind über eine (C₂-C₈)-Alkylenbrücke miteinander verbunden,
R⁵ ist H oder Methyl,
X ist O, NH, NR¹,
B: 0-99,9, vorzugsweise 80-99 Gew.-%
(Meth)acrylsäureester
C: 0-80, vorzugsweise 1-20 Gew.-% weiterer α,β-ungesättigter Verbindungen, wie HEMA oder EGDMA, gewonnen werden.

2. Verfahren nach Anspruch 1,
**dadurch gekennzeichnet, daß**
das polymervergrößerte Nitroxylderivat der Formel (II) 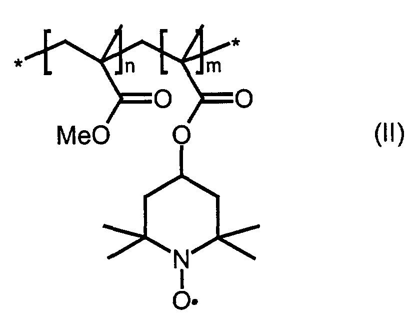 mit einem Verhältnis von n/m von 1-100 und einem mittleren Molekulargewicht von 1-200 kDa, vorzugsweise 10-100 kDa, entspricht.

3. Verfahren nach Anspruch 1 und/oder 2,
**dadurch gekennzeichnet, daß**
man als Oxidationsmittel NaOCl-Lösung verwendet.

4. Verfahren nach einem oder mehreren der Ansprüche 1 bis 3,
**dadurch gekennzeichnet, daß**
die Oxidation bei Temperaturen von -20 - 80°C, vorzugsweise 0 - 30°C, durchführt.

5. Verfahren nach einem oder mehreren der Ansprüche 1 bis 4,
**dadurch gekennzeichnet, daß**
man die Oxidation bei einem pH von 6-13, vorzugsweise 9-10, durchführt.

6. Verfahren nach einem oder mehreren der Ansprüche 1 bis 5,
**dadurch gekennzeichnet, daß**
man die Reaktion kontinuierlich in einem Menbranreaktor durchführt.

7. Verwendung der nach einem oder mehreren der Ansprüche 1 bis 10 hergestellten Verbindungen in der organischen Synthese.
